Europäisches Patentamt

European Patent Office

Office européen des brevets

(11) Publication number: **0 273 268**
**A2**

# EUROPEAN PATENT APPLICATION

(21) Application number: 87118331.5

(22) Date of filing: 10.12.87

(51) Int. Cl.4: **C12N 9/28** , C12N 9/96 ,
C12N 9/99 , A21D 8/04

(30) Priority: 22.12.86 US 944129

(43) Date of publication of application:
06.07.88 Bulletin 88/27

(84) Designated Contracting States:
BE CH DE ES FR GB IT LI NL

(71) Applicant: ENZYME BIO-SYSTEMS LTD.
International Plaza P.O. Box 8000
Englewood Cliffs, N.J. 07632(US)

(72) Inventor: Brumm Phillip J.
7921 S. Artesian
Chicago, Illinois 60652(US)

(74) Representative: Lederer, Franz, Dr. et al
Van der Werth, Lederer & Riederer
Patentanwälte Lucile-Grahn-Strasse 22
D-8000 München 80(DE)

(54) Reduced-stability alpha-amylase and process for its production.

(57) A modified bacterial alpha-amylase enzyme composition and method for its production by acylating an alpha-amylase enzyme, particularly, the alpha-amylase enzyme produced by Bacillus stearothermophilus . These enzyme compositions are useful as adjuncts to flour used for baking purposes.

FIG.1

EFFECT OF TEMPERATURE ON ALPHA-AMYLASE
ACTIVITY AT pH 5.5

EP 0 273 268 A2

# REDUCED-STABILITY ALPHA-AMYLASE AND PROCESS FOR ITS PRODUCTION

This invention relates to modified bacterial alpha-amylase enzyme compositions having reduced thermostability, and to a method for their preparation. Such compositions are particularly useful as adjuncts to flour used for baking purposes.

When wheat flour is used in baking and related industries, it is customary to add additional material which contains alpha-amylase enzymes. This is done to give the flour a minimum amount of alpha-amylase for consistency in baking. The amylase commonly used for this purpose is a malt amylase product.

Although the malt amylases have been used extensively in the baking industry, there are certain disadvantages to their use. Such amylases frequently impart an undesired color to the baked products. Furthermore, the malt preparations often contain a number of other enzymes, some of which may cause undesirable side reactions in the baking process. Accordingly, it would be desirable to find other alpha-amylase enzyme preparations which do not have these drawbacks.

When alpha-amylases of fungal origin have been tested as a replacement for malt amylases, they have proven to be too easily destroyed by the heat to make them useful for this purpose. On the other hand, when bacterial alpha-amylase preparations have been tested, they have been found to be too thermostable for this use. Such thermostable enzymes survive the baking process and continue to hydrolyze starchy materials after the baking process is completed giving rise to undesirable pasty products. These problems are discussed in detail in U.S. Patent 4,320,151.

When Hora acylated the bacterial alpha -amylase produced by B. subtilis (Bacillus subtilis) with. N-acetoxysuccinimide, he obtained modified alpha-amylases that were even more thermostable than the unmodified enzyme. Hora, J., Biochim. Biophys. Acta, 310, 264-267 (1973). This makes such enzymes even less suitable for use in baking processes.

We have now discovered, to our surprise, that certain bacterial alpha-amylases can be acylated in a manner which makes them less thermostable. The resulting modified alpha-amylases are particularly suitable for use in the baking industry or wherever a relatively pure alpha-amylase of reduced thermostability is required. Furthermore, they do not suffer from the drawbacks associated with the malt and other alpha-amylases previously used in baking and related industries.

In accordance with the present invention, there is provided a modified bacterial alpha-amylase enzyme having substantially less thermostability at 90°C than does the corresponding unmodified bacterial alpha-amylase at 90°C. The modified bacterial alpha-amylase comprises an acyl derivative of the alpha-amylase enzyme derived from B. stearothermophilus (Bacillus stearothermophilus) or B. subtilis.

Also provided, in accordance with this invention, is a process for the preparation of a modified bacterial alpha-amylase enzyme having reduced thermostability. This process comprises forming an acyl derivative of the alpha-amylase enzyme in the presence of a starch or starch hydrolyzate until at least about 50% of the free amino groups in the enzyme have been acylated.

Further, in accordance with this invention, is provided a flour standardized with an alpha-amylase enzyme to an alpha-amylase level of from about 0.4 to about 0.6 SKB units per gram of flour. The alpha -amylase enzyme is a modified bacterial alpha-amylase enzyme comprising an acyl derivative of the alpha-amylase derived from B. stearothermophilus.

FIG. 1 shows the relationship between the temperature and enzyme activity at pH 5.5 for a modified alpha-amylase of the present invention, and a similar relationship for unmodified alpha-amylase.

FIG. 2 shows the relationship between the temperature and enzyme activity at pH 4.5 for a modified alpha-amylase of the present invention, and a similar relationship for the unmodified alpha-amylase.

The alpha-amylase produced by the microorganism, B. stearothermophilus, is a preferred starting material for the preparation of the modified bacterial alpha-amylases of this invention. The alpha-amylase of B. stearothermophilus and methods for its preparation are given in U.S. Patents 4,284,722 and 4,493,893. A commercial B. stearothermophilus alpha-amylase enzyme is available from the Enzyme Development Corp., 2 Penn Plaza, New York, New York.

Another alpha-amylase which may be used in the practice of this invention is the alpha-amylase available from the Sigma Chemical Co., St. Louis, Missouri. This enzyme is listed as B subtilis amylase, but there is a suggestion that the producing microorganism may be the closely-related B. amyloliquefaciens - (Bacillus amyloliquefaciens).

Concentrations of alpha-amylase enzyme solutions were determined by the following assay method. The solution to be analyzed is diluted with 2.5 mM calcium chloride solution to give a final concentration of about 0.25 unit of activity per ml. One ml of properly diluted enzyme solution is added to 10 ml of a 1% soluble starch solution containing 0.03 M acetic acid buffer (pH 6.0) and 0.03 M calcium chloride. The reaction is carried out for 10 minutes at 60°C. One ml of the reaction solution is put in a 100-ml graduated flask containing 50 ml of 0.02 N hydrochloric acid, and after adding 3 ml of 0.05% iodine solution thereto, the total volume is made up to 100 ml by the addition of water. The blue color which develops is measured for absorbance at 620 nm. The amount of the enzyme required to decompose 10 mg starch in 1 minute is defined as 1 unit. (One unit of B. stearothermophilus so defined equals 0.8 SKB unit used in the baking industry.)

$$1 \text{ unit} = \frac{D_0 - D_s}{D_0} \times \frac{50}{10 \times 10} \times (\text{dilution factor})$$

where,

$D_0$ = absorbance of control solution (water is added instead of the enzyme solution)

$D_s$ = absorbance of the reaction solution

The modified alpha-amylases of this invention are prepared by acylating the free amino groups of the alpha-amylases. Acylation is accomplished by the use of an acylating agent that will react with the free amino groups without reacting with other functional groups within the enzyme molecule.

The preferred acylating agents used in the process of this invention are the anhydrides of monocarboxylic acids. Acetic anhydride is one preferred reagent. Other useful reagents are propionic, butyric, valeric, and benzoic anhydrides. Cyclic anhydrides of dicarboxylic acids are generally less suitable for this process, although, succinic anhydride does give a modified alpha-amylase of acceptably reduced thermostability.

The amount of acylating agent used in the process of this invention is not critical. However, sufficient reagent is used to acylate at least about 50% of the free amino groups in the enzyme.

In the practice of this invention, the unmodified alpha -amylase enzyme is first mixed with a starch or a low D.E. (dextrose equivalent) starch hydrolyzate in order to protect the enzyme from denaturation during the acylation reaction. For this purpose, it is convenient to carry out the reaction in a solution which contains about 10% of a water-soluble 10 D.E. starch hydrolyzate.

When the acylation reaction is carried out using acid anhydrides, the reaction is carried out at a temperature below that at which the enzyme is inactivated. A useful temperature range is from about 5°C to about 50°C for this purpose. A preferred temperature range is from about 25°C to about 30°C.

The acylation reaction with acid anhydrides is normally carried out at a pH above 7. The preferred pH range for this reaction is between about 8 and 9.

The modified bacterial alpha-amylase enzyme compositions of this invention are particularly useful as adjuncts to flour used for baking purposes. When used in this application, a sufficient amount of the modified alpha -amylase is added to give a standardized flour with the desired alpha-amylase level of activity. This is usually from about 0.4 to about 0.6 SKB unit, preferably about 0.5 SKB unit, per gram of flour. (An SKB unit is a measure of alpha-amylase activity employed in the baking industry. As noted above, 1 unit of B. stearothermophilus alpha-amylase enzyme activity, as measured by the test given herein, is equal to about 0.8 SKB unit.)

Furthermore, when flour is standardized using the modified bacterial alpha-amylase of the present invention, it shows the same type of reduction in Brabender viscosity as does the previously-used malt alpha-amylase. This permits evaluation of the standardized flour samples by means of standard Brabender viscosity procedures used in the bakery industry.

A flour standardized with the modified bacterial alpha-amylase enzyme of the present invention may be used for making various bakery products. Such products have improved storage stability showing good antistaling properties.

The following examples illustrate the invention. It is to be understood that the examples are illustrative only and do not intend to limit the invention in any way. In the examples, all parts and percentages are by weight, unless otherwise indicated, and all temperatures are degrees centigrade.

## EXAMPLE 1

The commercial alpha-amylase enzyme produced by B. stearothermophilus, available as G-ZYME 995 from the Enzyme Development Corp., 2 Penn Plaza, New York, New York, was purified by means of starch affinity using the following procedure.

Corn starch was used to adsorb selectively the alpha-amylase. The starch was first soaked in 0.05 M sodium acetate, 0.5 mM calcium chloride at pH 6.0 for several days at 5°C. The buffer was decanted and replaced with fresh, room temperature buffer. About 270,000 units of enzyme was added to 300 g of starch in a total volume of 2 liters of the above buffer. This suspension was stirred for 30 minutes at 5°C. The suspension was centrifuged at 4080 × g for 15 minutes at 5°C. The pellet was resuspended in fresh, cold buffer and recentrifuged. This washing procedure was repeated twice. The final pellet was resuspended in fresh buffer (1500 ml). The vessel containing this suspension was then immersed in a 60°C bath and stirred. When the temperature reached 54°C in the vessel, the hydrolysis was allowed to proceed 40 minutes. The vessel was at 60°C for about 30 minutes. The suspension was centrifuged at 16,300 × g for 15 minutes at 5°C. The pellet was discarded. The straw yellow supernatant contained the alpha-amylase enzyme.

A 20 ml solution of the purified alpha-amylase enzyme containing about 10,000 units of the enzyme was adjusted to pH 8.5 with dilute sodium hydroxide solution. Then, 2 grams of a 10 D.E. corn starch hydrolyzate was dissolved in the enzyme solution. To the starch solution was slowly added 0.2 g of the anhydride. The anhydride was added in portions over a period of 30 minutes. Dilute sodium hydroxide was added simultaneously with the anhydride to maintain the pH above 8.0. The temperature was maintained at 25°-27°C during the acylation reaction. After 30 minutes, the pH of the solution was adjusted to 6.8 with dilute hydrochloric acid. The enzyme solution may be used as is or further purified by conventional methods, such as dialysis or ultrafiltration.

The extent of modification of the enzyme was determined by measuring the residual-free amino groups in the enzyme using 2,4,6-trinitrobenzenesulfonic acid reagent following the analytical method of Habeeb, Analytical Biochemistry, 14, 328-336 (1966). A similar determination on purified, unmodified alpha-amylase enzyme was used as a reference standard.

The thermostabilities of the acylated enzyme were determined by measuring the enzyme activity using the standard alpha-amylase enzyme activity test at 60°C and comparing this value with the enzyme activity obtained when the test was run at 90°C. The results of these tests are given in Table I. They show that a significant percentage of the free amino groups have reacted with the anhydride and that the resulting acyl derivatives have considerably reduced thermostability over that of the unmodified enzyme.

### TABLE I

#### ACYLATED B. STEAROTHERMOPHILUS ALPHA-AMYLASE ENZYME

| Anhydride | Enzyme Activity, 90°C/ Enzyme Activity, 60°C | Free Amino Group Acylated (%) |
|---|---|---|
| None (Comparative Test) | 1.3 | 0 |
| Acetic | 0.40 | 73 |
| Propionic | 0.39 | 76 |
| Butyric | 0.76 | 95 |
| Valeric | 1.02 | 70 |
| Succinic | 0.88 | 63 |
| Benzoic | 0.97 | 25 |

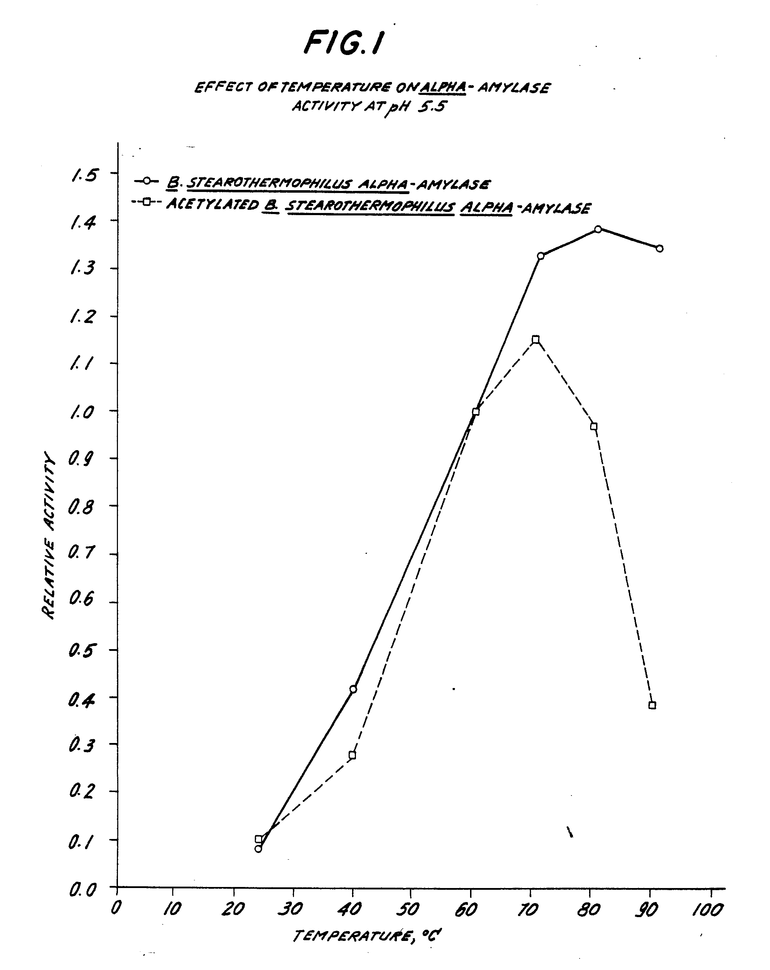

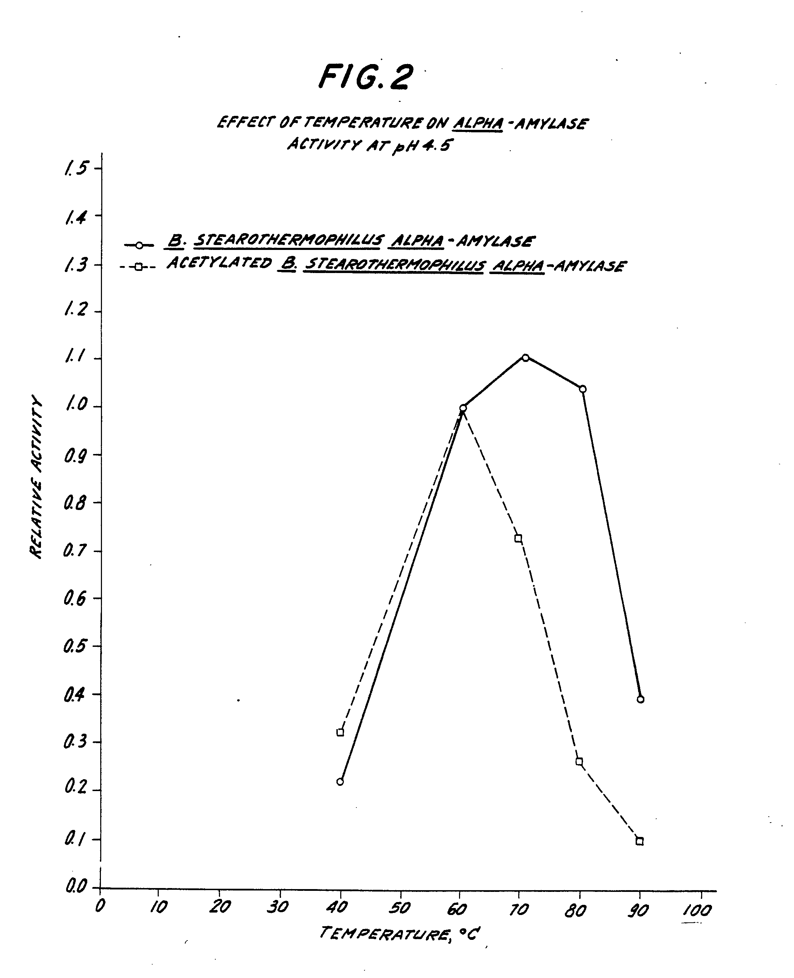

The activity of the acetylated B. stearothermophilus alpha-amylase enzyme was compared with that of the unmodified enzyme over a range of temperatures at pH 4.5 and 5.5. For comparison purposes, samples were normalized to give identical activities at 60°C. Activities were measured using standard test conditions except that the temperatures and pHs were varied. Results of these comparisons are given in FIG. 1 and FIG. 2. They show that the acetylated B. stearothermophilus alpha-amylase is much less thermostable than the unmodified enzyme.

EXAMPLE 2

Crystalline bacterial alpha-amylase (Sigma A 6380), listed as coming from B. subtilis, was acylated with acetic anhydride in the presence of a 10 D.E. corn starch hydrolyzate in the same manner as used for the acetylation of the alpha -amylase enzyme from B. stearothermophilusin Example 1. The thermostability of the acylated enzyme was determined by measuring the enzyme activity using the standard alpha-amylase enzyme activity test at 60°C and comparing this value with the enzyme activity obtained when the test was run at 80°C. While the ratio of enzyme activity at 80°C to enzyme activity at 60°C for the unmodified enzyme was 1.3, the ratio of these activities for the acylated enzyme was 0.5. The corresponding ratios for the unmodified enzyme and the acetylated enzyme at 90°:60°C were 0.82 and 0.28, respectively. This indicates that the acetylation carried out by the process of this invention results in a considerable decrease in thermostability of the B. subtilis enzyme. However, the acylated enzyme showed only about 10% of the activity of the starting material. This indicates that the process causes some inactivation of the enzyme.

Comparative Test

The general procedure of Example 1 was repeated using TERMAMYL, a commercial of amylase enzyme from B. licheniformis (Bacillus licheniformis), available from Novo Laboratories, Wilton, Connecticut. The enzyme was purified by means of starch affinity before it was acetylated with acetic anhydride. Acetylation caused no appreciable loss of activity of this enzyme. The degree of acetylation of the amylase from B. licheniformis was approximately the same as that of the amylase from B. stearothermophilus as shown by gel electrophoresis. However, this method of acetylation of the enzyme failed to reduce the stability of the enzyme to any appreciable degree.

These results indicate that the process of this invention is suitable for reducing the thermostability of select alpha-amylase enzymes and is particularly applicable to reducing the thermostability of the alpha -amylase enzyme from B. stearothermophilus.

**Claims**

1. A modified bacterial alpha-amylase enzyme having substantially less thermostability at 90°C than does the corresponding unmodified bacterial alpha-amylase at 90°C, said modified bacterial alpha-amylase comprising an acyl derivative of the alpha-amylase enzyme derived from Bacillus stearothermophilus or Bacillus subtilis.

2. The modified bacterial alpha -amylase of claim 1 wherein the acyl derivative is selected from the group: acetyl, propionyl, butyryl, valeryl, succinyl, and benzoyl.

3. A process for the preparation of a modified bacterial alpha -amylase enzyme having reduced thermostability which comprises forming an acyl derivative of the alpha-amylase enzyme in the presence of a starch or starch hydrolyzate until at least about 50% of the free amino groups in the enzyme have been acylated.

4. The process of claim 3 wherein the alpha-amylase enzyme is derived from Bacillus stearothermophilus or Bacillus subtilis.

5. The method of claim 4 wherein the acyl derivative is selected from the group: acetyl, propionyl, butyryl, valeryl, succinyl, and benzoyl.

6. The method of claim 4 wherein the acylation is accomplished by reacting the enzyme with an acid anhydride at a pH between about 7 and about 9 at a temperature between about 5°C and about 50°C.

7. The method of claim 6 wherein the acid anhydride is selected from the group: acetic anhydride, propionic anhydride, butyric anhydride, valeric anhydride, succinic anhydride, and benzoic anhydride.

8. A flour standardized with an alpha-amylase enzyme to an alpha-amylase level of from about 0.4 to about 0.6 SKB unit per gram of flour, wherein the alpha-amylase enzyme is a modified bacterial alpha-amylase enzyme comprising an acyl derivative of the alpha-amylase derived from Bacillus stearothermophilus or Bacillus subtilis.

9. The flour of claim 8 wherein the flour is standardized to an alpha-amylase level of about 0.5 SKB unit per gram of flour.

10. A method for making a bakery product having improved storage stability which comprises using as the flour ingredient in said bakery product a flour of claim 8.

# FIG.1

EFFECT OF TEMPERATURE ON <u>ALPHA</u>- AMYLASE
ACTIVITY AT pH 5.5

—o— <u>B.</u> STEAROTHERMOPHILUS ALPHA-AMYLASE
--□-- ACETYLATED <u>B. STEAROTHERMOPHILUS</u> <u>ALPHA</u>-AMYLASE

ENZYME BIO-SYSTEMS LTD.
3377

0 273 268

# FIG.2

EFFECT OF TEMPERATURE ON <u>ALPHA</u> -AMYLASE
ACTIVITY AT pH 4.5

─○─ <u>B. STEAROTHERMOPHILUS</u> <u>ALPHA</u>-AMYLASE
--□-- ACETYLATED <u>B. STEAROTHERMOPHILUS</u> <u>ALPHA</u>-AMYLASE

RELATIVE ACTIVITY

TEMPERATURE, °C